# EUROPEAN PATENT SPECIFICATION

(11) **EP 1 860 090 B1**
(45) Date of publication and mention of the grant of the patent: **28.11.2012**
(21) Application number: 07104654.4
(22) Date of filing: 22.03.2007
(51) Int. Cl.: C07C 29/00, C07C 29/60, C07C 45/52

(54) **Method for processing the glycerol phase from transestrification of fatty acid triglycerols**
Verfahren zur Verarbeitung der Glycerolphase von der Transesterifikation von Fettsäuretriglycerolen
Procédé de traitement d'une phase de glycérol à partir de la transestérification des triglycérides d'acide gras

(30) Priority: 24.03.2006 PL 37928306
(43) Date of publication of application: 28.11.2007
(73) Proprietor: Instytut Chemii Przemyslowej im. Prof. Ignacego Moscickiego, 01-793 Warszawa (PL)
(72) Inventor: Kijenski, Jacek, 01-461, Warszawa (PL); Migdal, Antoni, 26-600, Radom (PL); Osawaru, Osazuwa, 02-626, Warszwa (PL); Smigiera, Ewa, 11-400, Ketrzyn (PL)
(74) Representative: Schwabe, Hans-Georg

(56) References cited:
- WO-A-2006/087084
- FR-A- 695 931
- US-A- 2 558 520
- US-A- 5 387 720
- US-A- 5 616 817

## Description

The object of this invention is a method for processing the glycerol phase from transestrification of fatty acid triglycerols. The purpose of processing this fraction is to convert glycerol, in particular to acroleine and allyl alcohol.

Patent literature cites conversion reactions of glycerol to acroleine and allyl alcohol. Patent description US 5,387,720 describes a glycerol conversion process from a water/glycerol solution containing 10-40% by weight glycerol at 353-613 K in presence of solid catalysts, with Hammet acidity Hₒ(-20)-2. Examples of catalysts given include impregnated systems obtained by soaking of aluminum oxide with phosphoric acid, oxide systems containing heretopolyacids, as well as zeolites. The maximal molar conversion of glycerol to acroleine was 70.5%. Reaction products identified included also 1-hydroxyacetone and 3-hydroxypropane.

Another example of glycerol dehydration has been presented in patent description FR 695931. Vapors of glycerol contact a catalyst bed at temperature above 573 K. The catalyst consists of trihydroxyl acid salts (e.g. phosphates) of lithium or iron. Molar conversion of glycerol to acroleine in this reaction was 75%.

Also known is a process of obtaining acroleine from glycerol in a process conducted in the liquid phase. Buchler, W., Dinjus, E., Ederer, H.J., Kruse, A., Mas. C. in J. Supercritical Fluids, 22, 37 (2002) describe a process of glycerol dehydration conducted at 622-748 K and 25-45 MPa. Glycerol concentration in feedstock was 0-0.5 mol.dm⁻³. Besides acroleine and allyl alcohol, identified reaction products included methanol, acetic aldehyde, propionic aldehyde, ethanol, formaldehyde, carbon monoxide, carbon dioxide and hydrogen. Glycerol conversion to acroleine in this process depended on temperature and pressure, reaching maximally 24% (623 K, 45 MPa), to allyl alcohol 40% (745 K, 45 MPa and 667 K, 25 MPa). Formaldehyde and acetic aldehyde were the dominating liquid products in the post-reaction mix. Conversion of glycerol to these compounds was 40-80%.

Anatal Jr., M.J., Mok, W. S.L., Roy, J.C., Raissi, A.T. in J. Analyt. Appl. Pyrolysis, 8, 291 (1985) describe a glycerol dehydration process in subcrtitical water containing sulfuric acid as a catalyst. Glycerol conversion at 34 MPa and 623 K was 40% maximum. Glycerol concentration in the feedstock was 5% by weight.

Known is a method of glycerol conversion to acroleine described by Adkins, H. and Hartung, W.H. in Org. Synth. Coll. 1, 15 (1941). Pure anhydrous glycerol reacts with potassium bisulfite in presence of potassium sulfate at approx. 473 K. Product of this reaction contains primarily acroleine and is subsequently purified. The final molar conversion to acroleine is 33-48%.

Also known is a process of glycerol conversion to allyl alcohol conducted in the liquid phase, described by Kamm, O. and Marvel, C.S. in Org. Synth., Coll. 1, 42 (1941). Pure anhydrous glycerol is introduced into a flask containing anhydrous formic acid. A sequence of reactions followed by removal of impurities leads to pure ally alcohol. Glycerol conversion is approx. 50%. Remainder in the flask contains considerable quantities of glycerol condensation products.

It was concluded that valuable products, i.e. acroleine and allyl alcohol could be efficiently obtained from the waste glycerol fraction being a side product of fatty acid triglycerols transestrification.

Method of processing the glycerol fraction from fatty acid triglycerols transestrification at temperature above 573 K in presence of an acid catalyst according to the invention consists in that the glycerol fraction in the form of a water solution containing in excess of 40% by weight glycerol and up to 10% by weight of transestrification side products, is introduced into a neutral, high boiling point liquid heated to temperature above 573 K and below the liquid boiling temperature, and the resulting glycerol containing vapors are contacted, possibly in a neutral gas stream, with a heterogeneous acid catalyst having redox properties at temperatures from 520 K to 640 K, with catalyst load calculated in respect to the glycerol fraction is maintained at below 50 g_{(glycerol phase)}· g_{(catalyst})⁻¹.h⁻¹.

The process according to the invention is advantageously conducted using a glycerol fraction containing 70-80% by weight of glycerol and possibly up to 5% by weight of transestrification process side products.

The neutral, high boiling point liquid is advantageously either synthetic or mineral oil or silicon oil.

It is advantageous to introduce the glycerol fraction into the high boiling point liquid heated to 600-650 K.

The neutral carrier gas is advantageously nitrogen or helium.

The heterogeneous acid catalyst having redox properties is advantageously a mixture containing sulfonated amorphous aluminosilicate, having 89% by weight content of SiO₂ and iron(III) oxide.

It is advantageous to conduct the process at catalyst loads below 10 g_{(glycerol phase)}· g_{(catalyst)}⁻¹.h⁻¹.

Raw material processed using the method according to the invention contains besides glycerol also other side products of the fat transestrification process, such as: sodium or potassium oxide, sodium or potassium carbonate, sodium or potassium soaps, resulting from neutralization of the transestrification process catalyst, as well as small quantities of fatty acids, fatty acid mono- and di-glycerols with boiling temperatures above 623 K.

Processing of the byproduct glycerol fraction from the fat transestrification (triglycerols) using the method according to the invention can lead to an overall conversion of glycerol to desired products, i.e. acroleine and allyl alcohol, at levels of up to 70% relative to glycerol contained in the substrate.

Processing of the glycerol fraction obtained from the transestrification of fatty acid triglycerols process according to the invention has been illustrated on examples.

### Example I.

Glycerol fraction from the transestrification of fatty acid triglycerols with methanol, containing 75% by weight glycerol, 3% by weight of transestrification byproducts (primarily potassium chloride and fatty acids) was added to 100 cm3 of high boiling point liquid - silicon oil (Polsil OM-350) at approx. 603 K. Vapors of the evaporated liquid were forced with stream of nitrogen flowing at 120 cm³.h⁻¹ though a catalyst bed consisting of a mixture of sulfonated amorphous aluminosilicate containing 89% by weight of Si02 and iron(III) oxide. The catalyst, calcinated for 1 hour at 623 K in a stream of nitrogen at 573 K had a grain size range of 0.5--1.5 mm. Catalyst load calculated per the glycerol fraction was 1.3 g_{(glyccrol phase)}· g_{(catalyst)}⁻¹.h⁻¹. Overall glycerol conversion was 100%, molar conversion to acroleine was 35%, to allyl alcohol 28%, and to methanol 7%. These values were maintained for 4 hours of continuing the process. Reaction products, i.e. acroleine and allyl alcohol were separated taking advantage of their boiling temperature difference.

### Example II.

The process was conducted as provided for in Example I, only temperature of the high boiling point liquid - mineral oil , in which the water/glycerol solution was evaporated was 623 K, with catalyst load of 5.7 g_{(glycerol phase)}· g_{(catalyst)}⁻¹.h⁻¹. Total glycerol conversion was 87%, molar conversion to acroleine was 28%, to allyl alcohol 26% and to methanol 8%.

### Example III.

The process was conducted as provided for in Example I, only the catalyst used was sulfonated titanium(IV) oxide with grain size 0.5-1.5 mm and helium was the carrier gas. Catalyst load of 5.7 g_{(glycerol phase)}· g_{(catalyst)}⁻¹.h⁻¹. Total glycerol conversion was 98%, molar conversion to acroleine was 34%, to allyl alcohol 12% and to methanol 17%.

### Example IV.

The process was conducted as provided for in Example I, only the substrate used was a glycerol fraction from transestrification of fatty acid triglycerols with methanol, containing 63% by weight glycerol, 7% by weight of other transestrification byproducts (principally potassium chloride and potassium soaps) and water. Total glycerol conversion was 100%, molar conversion to acroleine was 30%, to allyl alcohol 28% and to methanol 10%.

### Example V.

The process was conducted as provided for in Example I, only the catalyst bed temperature was 533 K, temperature of the high boiling point liquid - synthetic oil based on poly(alpha-olefins) - in which the glycerol fraction was evaporated, was 583 K. Total glycerol conversion was 100%, molar conversion to acroleine was 35%, to allyl alcohol 31% and to methanol 9%.

### Example VI.

The process was conducted as provided for in Example I, only the carrier gas used was helium and the high boiling point liquid - synthetic oil based on poly(alphaolefins) - in which the glycerol fraction was evaporated, was additionally heated during the reaction with a microwave field to 603 K. Total glycerol conversion was 100%, molar conversion to acroleine was 35%, to allyl alcohol 30% and to methanol 8%.

### Example VII.

The process was conducted as provided for in Example I, only the catalyst load was 10.4 g_{(glycerol phase)}· g_{(catalyst)}-¹.h⁻¹, and nitrogen stream flow rate 318 cm3.h-1. Total glycerol conversion was 75%, molar conversion to acroleine was 21%, to allyl alcohol 18% and to methanol 13%.

## Claims

1. Method for processing the glycerol phase from transesterification of fatty acid triglycerols at temperature above 573 K in presence of an acid catalyst, **significant in that** the glycerol fraction in the form of a water solution containing more than 40% by weight of glycerol and up to 10% by weight of transesterification byproducts, is introduced into a neutral high boiling point liquid heated to temperature above 573 K and below its boiling temperature, with the evolving vapors containing glycerol are subsequently contacted with a heterogeneous acid catalyst having redox properties, at temperatures of 520-640 K, with catalyst load calculated in respect to the glycerol fraction maintained below 50 g_{(glycerol phase)} g_{(catalyst)}⁻¹.h⁻¹, wherein acroleine and allyl alcohol are obtained.

2. Method according to Claim 1, **significant in that** a glycerol fraction containing 70-80% by weight of glycerol and possibly up to 5% by weight of transestrification byproducts is used.

3. Method according to Claim 1, **significant in** that the high boiling point liquid used is mineral or synthetic oil or silicone oil.

4. Method according to Claim 1, **significant in that** the glycerol fraction is introduced into the high boiling point liquid heated to 600-650 K.

5. Method according to Claim 1, **significant in that** the contact of the vapors containing glycerol and the heterogenous acid catalyst is carried out in a neutral gas stream.

6. Method according to Claim 5, **significant in that** the neutral carrier gas used is nitrogen or helium.

7. Method according to Claim 1, **significant in that** the heterogeneous acid catalyst having redox properties used comprises a mixture containing sulfonated amorphous aluminosilicate with 89% by weight of SiO₂ and iron(III) oxide.

8. Method according to Claim 1, **significant in that** the heterogeneous acid catalyst having redox properties used comprises sulfonated titanium(IV) oxide.

9. Method according to Claim 1, **significant in that** the process is conducted at catalyst loads below 10 g_{(glycerol phase)} g_{(catalyst)} .h⁻¹.

## Patentansprüche

1. Verfahren zur Verarbeitung der Glycerinphase aus der Umesterung von Fettsäuretriglyceriden bei einer Temperatur höher als 573 K in Gegenwart eines sauren Katalysators, **dadurch gekennzeichnet, dass** die Glycerinfraktion in der Form einer Wasserlösung, die mehr als 40 Gew.-% Glycerin und bis zu 10 Gew.-% Umesterungsnebenprodukte enthält, in eine neutrale hochsiedende, auf eine Temperatur oberhalb von 573 K und unterhalb ihrer Siedetemperatur erwärmte Flüssigkeit eingebracht wird, wobei die sich entwickelnden Dämpfe, die Glycerin enthalten, anschließend mit einem heterogenen sauren Katalysator mit Redoxeigenschaften bei Temperaturen von 520-640 K kontaktiert werden, wobei die Katalysatorbeladung, die bezüglich der Glycerinfraktion berechnet wird, unterhalb von 50 g_{(Glycerinphase)} g_{(Katalysator)}⁻¹.h⁻¹ gehalten wird, wobei Acrolein und Allylalkohol erhalten werden.

2. Verfahren nach Anspruch 1, **dadurch gekennzeichnet, dass** eine Glycerinfraktion, die 70 bis 80 Gew.-% Glycerin und möglicherweise bis zu 5 Gew.-% Umesterungsnebenprodukte enthält, verwendet wird.

3. Verfahren nach Anspruch 1, **dadurch gekennzeichnet, dass** die verwendete hochsiedende Flüssigkeit Mineralöl oder synthetisches Öl oder Silikonöl ist.

4. Verfahren nach Anspruch 1, **dadurch gekennzeichnet, dass** die Glycerinfraktion in die auf 600-650 K erwärmte hochsiedende Flüssigkeit eingebracht wird.

5. Verfahren nach Anspruch 1, **dadurch gekennzeichnet, dass** der Kontakt der Dämpfe, die Glycerin enthalten, und des heterogenen sauren Katalysators in einem neutralen Gasstrom durchgeführt wird.

6. Verfahren nach Anspruch 5, **dadurch gekennzeichnet, dass** das verwendete neutrale Trägergas Stickstoff oder Helium ist.

7. Verfahren nach Anspruch 1, **dadurch gekennzeichnet, dass** der verwendete heterogene saure Katalysator mit Redoxeigenschaften ein Gemisch umfasst, das sulfoniertes amorphes Aluminosilicat mit 89 Gew.-% SiO₂ und Eisen(III)-oxid enthält.

8. Verfahren nach Anspruch 1, **dadurch gekennzeichnet, dass** der verwendete heterogene saure Katalysator mit Redoxeigenschaften sulfoniertes Titan(IV)-oxid umfasst.

9. Verfahren nach Anspruch 1, **dadurch gekennzeichnet, dass** das Verfahren bei Katalysatorbeladungen unterhalb von 10 g_{(Glycerinphase)} g_{(Katalysator)}⁻¹.h⁻¹ durchgeführt wird.

## Revendications

1. Procédé de traitement d'une phase glycérol à partir de la transestérification des triglycérides d'acide gras à une température supérieure à 573 K en présence d'un catalyseur acide, **caractérisé en ce que** la fraction glycérol, sous forme d'une solution aqueuse contenant plus de 40 % en poids de glycérol et jusqu'à 10 % en poids de sous-produits de transestérification, est introduite dans un liquide neutre à point d'ébullition élevé chauffé à une température supérieure à 573 K et inférieure à sa température d'ébullition, dans lequel les vapeurs se dégageant contenant du glycérol sont ensuite mises en contact avec un catalyseur acide hétérogène qui a des propriétés rédox, à des températures de 520 à 640 K, dans lequel la charge de catalyseur calculée par rapport à la fraction glycérol est maintenue en-dessous de 50 g_{(phase glycérol)} g_{(catalyseur)}⁻¹.h⁻¹, dans lequel on obtient de l'acroléine et de l'alcool allylique.

2. Procédé selon la revendication 1, **caractérisé en ce qu'**une fraction glycérol contenant 70 à 80 % en poids de glycérol et possiblement jusqu'à 5 % en poids de sous-produits de transestérification est utilisée.

3. Procédé selon la revendication 1, **caractérisé en ce que** le liquide à point d'ébullition élevé utilisé est une huile minérale ou une huile synthétique ou de l'huile de silicone.

4. Procédé selon la revendication 1, **caractérisé en ce que** la fraction glycérol est introduite dans le liquide à point d'ébullition élevé chauffé à 600 - 650 K.

5. Procédé selon la revendication 1, **caractérisé en ce que** le contact entre les vapeurs contenant du glycérol et le catalyseur acide hétérogène est réalisé dans un flux de gaz neutre.

6. Procédé selon la revendication 5, **caractérisé en ce que** le gaz porteur neutre utilisé est de l'azote ou de l'hélium.

7. Procédé selon la revendication 1, **caractérisé en ce que** le catalyseur acide hétérogène ayant des propriétés rédox utilisé comprend un mélange contenant de l'aluminosilicate amorphe sulfonaté avec 89 % en poids de SiO₂ et d'oxyde de fer_{(III)}.

8. Procédé selon la revendication 1, **caractérisé en ce que** le catalyseur acide hétérogène ayant des propriétés rédox utilisé comprend de l'oxyde de titane_{(IV)} sulfonaté.

9. Procédé selon la revendication 1, **caractérisé en ce que** le procédé est réalisé avec des charges de catalyseur inférieures à 10 g_{(phase glycérol)} g_{(catalyseur)}⁻¹. h⁻¹·
